# EUROPÄISCHE PATENTSCHRIFT

(11) **EP 0 589 359 B1**
(45) Veröffentlichungstag und Bekanntmachung des Hinweises auf die Patenterteilung: **28.06.1995**
(21) Anmeldenummer: 93114921.5
(22) Anmeldetag: 16.09.1993
(51) Int. Cl.: C07C 43/303, C07C 41/48, C07D 321/06

(54) **Verfahren zur Herstellung von Ketenacetalen**
Process for the production of ketene acetals
Procédé pour la préparation d'acétals cétènes

(30) Priorität: 17.09.1992 DE 4231193
(43) Veröffentlichungstag der Anmeldung: 30.03.1994
(73) Patentinhaber: Wacker-Chemie GmbH, 81737 München (DE)
(72) Erfinder: Fleischmann, Gerald, Dr., D-84489 Burghausen (DE); Eck, Herbert, Dr., D-84489 Burghausen (DE); Petersen, Hermann, D-84489 Burghausen (DE); Pflaum, Siegfried, Dr., D-84508 Burgkirchen (DE)

(56) Entgegenhaltungen:
- EP-A- 0 095 182
- WO-A-92/14722
- US-A- 3 431 281
- US-A- 4 603 224
- HOUBEN-WEYL "Methoden der organischen Chemie" Band VII/4, 4. Auflage 1968, GEORG THIEME VERLAG, Stuttgart, Seiten 340-343
- CHEMICAL ABSTRACTS, Band 113, Nr. 18, 29. Oktober 1990, Columbus, Ohio, USA BAILEY, WILLIAM J. et al. "Recent advances in ring- -opening polymerization of cyclic ketene acetals" Seite 1, Spalte 2, Zusammenfassung-Nr. 153 070z

## Beschreibung

Die Erfindung betrifft ein Verfahren zur Herstellung von Ketenacetalen durch Dehydrohalogenierung von 2-Halogenaldehydacetalen.

Es ist bekannt, Ketenacetale aus den entsprechenden 2-Halogenaldehydacetalen, durch Abspaltung von Halogenwasserstoff mit Hilfe von Alkoholaten oder Aminbasen, zu gewinnen. In Houben-Weyl Band 7/4, 4. Auflage Stuttgart 1968, Seite 340, und W.J. Bailey, Ring-opening Polymerisation in: Comprehensive Polymer Science Vol. 3, Pergamon Press 1989, Seite 283 - 326, wird die Bromwasserstoffabspaltung mittels Kalium-tert.-butylat beschrieben. Aus C.A.113(18):153070 ist die Dehydrobromierung in Gegenwart von Kalium-tert.-butylat und eines Phasentransferkatalysators bekannt. Die Dehydrochlorierung von 2-Chloromethyl-1,3-dioxolan mittels Natrium oder Kalium in flüssigem Ammoniak ist in der US-A 3431281 beschrieben. Nachteilig bei diesen Verfahrensweisen ist, daß mit diesen Methoden zum einen nur die reaktionsfreudigeren, aber teureren 2-Bromaldehydacetale als Ausgangsverbindungen zu verwenden sind und zum anderen als Basen die, für die Synthese im technischen Maßstab, relativ teuren (Erd)alkalialkoholate oder in flüssigem Ammoniak gelösten Alkalimetalle eingesetzt werden können.

Gegenstand der WO-A 92/14722 ist ein Verfahren zur Dehydrohalogenierung von halogenierten, cyclischen Ketenacetalen wie 2-Chlormethyl-1,3-dioxepan mit Alkali- bzw. Erdalkalihydroxidverbindungen in Gegenwart eines inerten Alkohols wie 2-Butanol. Nachteilig bei dieser Verfahrensweise sind die geringen Ausbeuten (geringe Selektivität sowie Nebenprodukte durch wässrige Aufarbeitung) die mit diesem Verfahren, nach obendrein aufwendiger Reinigung des Rohprodukts durch fraktionierte Destillation in Gegenwart eines organischen Lösungsmittels und eines Amins, erhalten werden.

Es bestand daher die Aufgabe, ein Verfahren zur Verfügung zu stellen, mit dem Ketenacetale sowohl ausgehend von 2-Bromaldehydacetalen als auch ausgehend von den billigeren, aber reaktionsträgeren 2-Chloraldehydacetalen zugänglich werden. Weiter sollte bei der Synthese die Verwendung von relativ teuren Basen wie (Erd)alkalialkoholaten oder in flüssigem Ammoniak gelösten Alkalimetallen vermieden werden und das Ketenacetal in guten Ausbeuten, ohne aufwendige Reinigungsmaßnahmen, erhältlich sein.

Gegenstand der Erfindung ist ein Verfahren zur Herstellung von Ketenacetalen durch Dehydrohalogenierung von offenkettigen oder cyclischen 2-Halogenaldehydacetalen mit einem Alkali- oder Erdalkalihydroxid, dadurch gekennzeichnet, daß die Dehydrohalogenierung in einem zweiphasigen System, in Gegenwart einer Katalysatorkombination aus
a) einem Phasentransferkatalysator und
b) einer oder mehreren Verbindungen aus der Gruppe sekundäre Alkohole, tertiäre Alkohole, sekundäre Diole, tertiäre Diole durchgeführt wird.

Das Verfahren eignet sich zur Herstellung von Ketenacetalen der Formeln 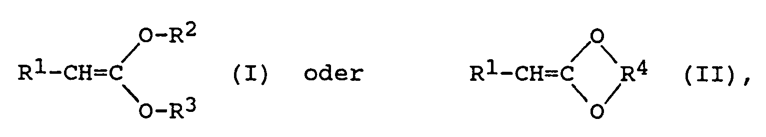
wobei R¹ Wasserstoff, einen geradkettigen, verzweigten oder cyclischen Alkylrest mit 1 bis 18, vorzugsweise 1 bis 4, C-Atomen, einen Alkenylrest, einen Arylrest oder Aralkylrest bedeuten kann, und
R² und R³ gleich oder verschieden sind und einen Alkylrest mit 1 bis 18, vorzugsweise 1 bis 4, C-Atomen, einen Alkenylrest, einen Arylrest oder Aralkylrest bedeuten können und
R⁴ einen Rest (CR⁵R⁶)ₘ mit m = 2 bis 5 bedeutet, wobei R⁵ und R⁶ gleich oder ungleich sein können und die Bedeutung Wasserstoff, geradkettiger oder verzweigter Alkylrest mit 1 bis 4 C-Atomen, Alkenylrest, Arylrest oder Aralkylrest haben.

Besonders bevorzugt sind Ketenacetale der obengenannten Formeln, bei denen R¹ Wasserstoff bedeutet, R² und R³ gleich sind und einen Alkylrest mit 1 bis 4 C-Atomen bedeuten und R⁴ einen -(CH₂)₄-Rest bedeutet.

Geeignete Edukte für das erfindungsgemäße Verfahren sind 2-Halogenaldehydacetale der Formeln 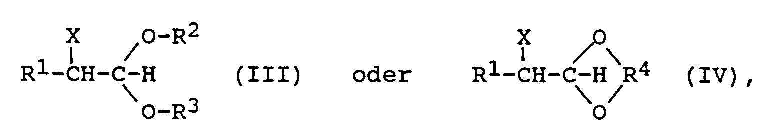
wobei R¹, R², R³ und R⁴ die obengenannten Bedeutungen haben können und X = Cl oder Br. Vorzugsweise werden Halogenaldehydacetale der Formeln (III) oder (IV) mit X = Cl eingesetzt. Insbesonders Halogenaldehydacetale der Formeln (III) oder (IV) mit X = Cl, wobei R¹ Wasserstoff bedeutet, R² und R³ gleich sind und einen Alkylrest mit 1 bis 4 C-Atomen bedeuten und R⁴ einen -(CH₂)₄-Rest bedeutet. Soweit die genannten 2-Halogenaldehydacetale nicht ohnehin im Handel erhältlich sind, lassen sich diese nach allgemein bekannten Synthesevorschriften herstellen: Ausgehend von den entsprechenden Aldehyden sind die 2-Halogenaldehyde durch säure- oder basenkatalysierte Halogenierung zugänglich. Mittels säurekatalysierter Umsetzung mit den entsprechenden Alkoholen erhält man die 2-Halogenaldehydacetale.

Die Dehydrohalogenierung wird in Gegenwart von Alkali- oder Erdalkalihydroxiden wie LiOH, NaOH, KOH, CsOH, Ca(OH)₂, Sr(OH)₂, Ba(OH)₂ durchgeführt. Vorzugsweise wird zur Dehydrohalogenierung Kaliumhydroxid eingesetzt. Das Molverhältnis von 2-Halogenaldehydacetal zu (Erd)alkalihydroxid beträgt von 1 : 1 bis 1 : 25, vorzugsweise 1 : 1 bis 1 : 5, besonders bevorzugt 1 : 2 bis 1 : 4.

Für das erfindungsgemäße Verfahren geeignete Phasentransferkatalysatoren sind beispielsweise Polyethylenglykol-dialkylether, Polyethylenglykol-aryl-aralkylether wie Triton^{R} CF10, Polyethylenglykol-alkyl-arylether, Kronenether, Tris-[2-(2-methoxyethoxy)ethyl]-amin, Tetraalkylammoniumsalze, Aralkyltrialkylammoniumsalze. Vorzugsweise verwendet werden Polyethylenglykol-dialkylether, Triton^{R} CF10 und Tris-[2-(2-methoxyethoxy)ethyl]-amin (TDA1). Die Phasentransferkatalysatoren werden dabei in einem Gewichtsverhältnis von Phasentransferkatalysator zu 2-Halogenaldehydacetal von 0.001 : 1 bis 1 : 1, vorzugsweise von 0.01 : 1 bis 0.2 : 1, eingesetzt.

Für das erfindungsgemäße Verfahren geeignete sekundäre oder tertiäre Alkohole sowie sekundäre oder tertiäre Diole sind beispielsweise Diphenylmethanol, t-Butanol, sec-Butanol, 2-Methyl-2-butanol, 3,3-Dimethyl-2-butanol, 3-Methyl-3-pentanol, 2,4-Dimethyl-3-pentanol, 2,4-Dimethylpentan-2,4-diol, 2,6-Dimethylcyclohexanol, 2,5-Dimethylhexan-2,5-diol. Die genannten Alkohole bzw. Diole werden in einem Molverhältnis von Alkohol bzw. Diol zu 2-Halogenaldehydacetal von 0.01 : 1 bis 20 : 1, vorzugsweise von 0.01 : 1 bis 2.5 : 1, besonders bevorzugt 0.01 : 1 bis 0.3 :1 eingesetzt.

Mit dem kombinierten Einsatz von Phasentransferkatalysator und sekundärer bzw. tertiärer Alkohol- oder Diolkomponente wird die Reaktion stark beschleunigt, so daß der Endumsatz wesentlich schneller als beim Einsatz nur einer der beiden Komponenten erreicht wird.

Das erfindungsgemäße Verfahren wird vorzugsweise in einem organischen, nichtalkoholischen Lösungsmittel durchgeführt. Als Lösungsmittel geeignet sind die in der organisch-chemischen Synthese üblicherweise verwendeten aliphatischen Kohlenwasserstoffe wie n-Hexan, halogenierte aliphatische Kohlenwasserstoffe wie Methylenchlorid, aromatische Kohlenwasserstoffe wie Toluol, halogenierte Aromaten wie Chlorbenzol oder Ether wie t-Butylmethylether, Diethylether, THF.

Das erfindungsgemäße Verfahren wird unter Normaldruck bei Temperaturen von 0 bis 200°C, vorzugsweise von 20 bis 130°C, besonders bevorzugt 20 bis 80°C, durchgeführt. Arbeiten unter Schutzgas ist nicht notwendig. Das Verfahren kann in den für die chemische Synthese üblichen Reaktionsapparaturen, welche mit Rührer, Rückflußkühler, Heizung/Kühlung und gegebenenfalls Wasserabscheider ausgerüstet sind, durchgeführt werden. Die im Verfahren zu verwendenden Edukte wie 2-Halogenaldehydacetal, Phasentransferkatalysator, tertiärer Alkohol bzw. Diol und (Erd)alkali-hydroxid können in technischer Qualität ohne weitere Vorbehandlung eingesetzt werden.

Das erfindungsgemäße Verfahren kann unter Vorlage aller oder einzelner Bestandteile des Reaktionsgemisches oder unter teilweiser Vorlage und Nachdosierung der oder einzelner Bestandteile des Reaktionsgemisches durchgeführt werden. Im allgemeinen wird das 2-Halogenaldehydacetal im Lösungsmittel vorgelegt, wobei eine Umsetzung ohne Lösungsmittel auch möglich ist. Die (Erd)alkalihydroxidkomponente sowie die Katalysatorkomponente (Phasentransferkatalysator und sekundärer bzw. tertiärer Alkohol (Diol)) werden im allgemeinen vorgelegt, können aber auch dosiert werden. Entscheidend für die Reaktion ist eine gute Durchmischung des zweiphasigen Systems, die durch kräftiges Rühren, vorzugsweise mit einer Rührgeschwindigkeit von 300 bis 1000 rpm, mit einem geeigneten Rührer, beispielsweise einem Propeller- oder Blattrührer, erreicht wird.

Das bei dem erfindungsgemäßen Verfahren als Nebenprodukt entstehende Wasser, welches mit dem gebildeten Ketenacetal zu Ester und Alkohol weiterreagieren würde, Kann durch kontinuierliche azeotrope Destillation mit einem geeigneten Lösungsmittel abdestilliert werden. Wird das (Erd)alkalihydroxid im Überschuß eingesetzt, wird das Reaktionswasser durch die Hydroxidkomponente absorbiert. Auf die azeotrope Destillation kann in diesem Fall verzichtet werden.

Die mit dem erfindungsgemäßen Verfahren herstellbaren Ketenacetale dienen als Zwischenprodukte in der organischen Synthese, beispielsweise als En-Komponente für Diels-Alder-Reaktionen. Cyclische Ketenacetale, insbesonders 2-Methylen-1,3-dioxepan, sind als Monomere oder Comonomere für radikalische Polymerisationen geeignet. 2-Methylen-1,3-dioxepan geht dabei eine Ringöffnungspolymerisation ein, wobei ein Polyester entsteht oder bei Copolymerisation mit anderen Monomeren ein Estergruppen enthaltendes Copolymer. Aufgrund der enzymatisch spaltbaren Estergruppen im Polymerrückgrat sind cyclische Ketenacetale als (Co)monomer-Komponente für die Herstellung biologisch abbaubarer Kunststoffe geeignet.

Die nachfolgenden Beispiele dienen der weiteren Erläuterung der Erfindung.

### Beispiel 1: (Herstellung von 3-Methylen-1,3-dioxepan)

In einem 21-Dreihalskolben mit KPG-Rührer, Rückflußkühler und Thermometer wurde eine Mischung aus 75.3 g (0.5 mol) 2-Chlormethyl-1,3-dioxepan, 150 ml tert.-Butylmethylether, 4.0 g Polyethylenglykol-1000-dimethylether, 11 g (125 mmol) 2-Methyl-2-butanol und 105 g (1.14 mol) feingemahlenes Kaliumhydroxid (85 %ig) 3.0 Stunden unter Rühren und Rückfluß erhitzt. Nach Abkühlen wurden Salze und überschüssige Base abfiltriert und das Reaktionsgemisch anschließend über eine Vigreuxkolonne destilliert. Ausbeute 40.5 g (71 % d. T.).

### Beispiel 2: (Herstellung von 1,1-Diethoxyethen)

152.6 g (1 mol) Chloracetaldehyd-diethylacetal, 200 g (3 mol) Kaliumhydroxid (85 %ig), 8 g Polyethylenglykol-1000-dimethylether, 22.0 g (250 mmol) 2-Methyl-2-butanol und 300 ml tert.-Butyl-methylether wurden 4 Stunden unter Rühren und Rückfluß erhitzt. Nach beendeter Reaktion wurden die Salze abfiltriert und die Reaktionsmischung unter Normaldruck destilliert. Das Produkt siedete bei 125°C (100 kPa). Ausbeute 89.3 g (77 % d. T.).

### Vergleichsbeispiel 1:

In einem 21-Dreihalskolben mit KPG-Rührer, Rückflußkühler und Thermometer wurde eine Mischung aus 600 g (4 mol) 2-Chlormethyl-1,3-dioxepan, 660 g (10 mol) fein gemahlenes Kaliumhydroxid (85 %ig) und 500 ml tert.-Butylmethylether 10 Stunden unter kräftigem Rühren unter Rückfluß erhitzt. Nach Abkühlen wurden Salze und überschüssige Base abfiltriert und das Reaktionsgemisch destilliert.Die einzige Produkt enthaltende Fraktion ergab 18 g eines Gemisches aus 29 % 2-Methylen-1,3-dioxepan (= 5.2 g) und 71 % 2-Chlormethyl-1,3-dioxepan. Der Umsatz betrug somit 1.14 % der Theorie.

### Beispiel 3: Herstellung von 2-Methylen-1,3-dioxepan

361.5 g (2.4 mol) 2-Chlormethyl-1,3-dioxepan, 720 ml tert.-Butylmethylether, 40 g Polyethylenglycol-1000-dimethylether, 100 g (0.25 mol) 2-Methyl-2-butanol und 500 g (7.5 mol) technisches Kaliumhydroxyd (91 %) wurden unter Rühren zum Rückfluß erhitzt. Nach 6 Stunden Reaktionszeit, wurde die Reaktion abgebrochen, die Salze abfiltriert, das Lösungsmittel bei Normaldruck und das Produkt bei 5 kPa destilliert. Die Ausbeute an reinem 2-Methylen-1,3-dioxepan betrug 81.4 % der Theorie, mit dem in den Zwischenfraktionen enthaltenem Produkt betrug die Gesamtausbeute 96.3 % der Theorie.

### Beispiel 4: Herstellung von 1,1-Dipropoxyethen

270.8 g (1.5 mol) 1-Chlor-2,2-dipropoxyethan, 450 ml tert.-Butylmethylether, 19.8 g (0.22 mol) 2-Methyl-2-butanol, 12 g Polyethylenglycol-1000-dimethylether und 300 g (4.5 mol) technisches Kaliumhydroxyd (91 %) wurden unter Rühren 6 Stunden zum Rückfluß erhitzt. Die Reaktion wurde abgebrochen die Salze abfiltriert und das Produkt destilliert. Der Umsatz betrug 86.3 % der Theorie. Die Hauptfraktion enthielt 148.1 g (68.6 % der Theorie) reines 1,1-Dipropoxyethen (Kp. 70°C bei 1.2 kPa).

### Beispiel 5: Herstellung von 1,1-Dibutoxyethen

209 g (1 mol) 1-Chlor-2,2-dibutoxyethan, 300 ml tert.-Butylmethylether, 13.2 g (0.13 mol) 2-Methyl-2-butanol, 8 g Polyethylenglycol-1000-dimethylether und 200 g (3 mol) technisches Kaliumhydroxyd wurden 6 Stunden unter Rühren zum Rückfluß erhitzt. Die Reaktionsmischung wurde wie voranstehend beschreiben aufgearbeitet. Der Umsatz betrug 77.1 % der Theorie, die Ausbeute an Reinsubstanz 60.8 % der Theorie (Kp. 87-88°C, 1.5 kPa).

### Beispiel 6: Herstellung von 1,1-Diethoxyethen

610.4 g (4.0 mol) Chloracetaldehyd-diethylacetal, 800 g (12.0 mol) pulverisiertes technisches Kaliumhydroxyd, 32 g Polyethylenglycol-1000-dimethylether und 88 g (1.0 mol) 2-Methyl-2-butanol wurden in 1.2 l tert.-Butylmethylether unter Rühren zum Rückfluß erhitzt. Nach 6 Stunden wurde die Reaktion abgebrochen, die Salze abfiltriert und die Reaktionsmischung unter vermindertem Druck (5 kPa) destilliert. Es wurden 326 g (72.5 % der Theorie) 1,1-Diethoxyethen (Kp. 49°C/5 kPa) erhalten.

### Beispiel 7: Herstellung von 2-Methylen-1,3-dioxepan (Technikumsansatz)

In einem 800 l V4A-Rührwerk mit Rückflußkühler wurden 70 kg (464.7 mol) 2-Chlormethyl-1,3-dioxepan, 96.8 kg (1451 mol) technisches Kaliumhydroxyd (gepulvert), 7.8 kg Polyethylenglycol-1000-dimethylether und 19.4 kg (48.5 mol) 2-Methyl-2-butanol in 116 kg tert.-Butylmethylether 6 Stunden zum Rückfluß erhitzt. Nach Abkühlen wurde mit 164 kg tert.-Butylmethylether verdünnt und die Reaktionsmischung, durch Filtration über eine Druckfilternutsche, von Salzen und überschüssiger Base getrennt.
Die gaschromatographische Untersuchung der Reaktionsmischung zeigte einen fast quantitativen Umsatz an 2-Chlormethyl-1,3-dioxepan. Die Rohausbeute von 2-Methylen-1,3-dioxepan betrug 93.7 % der Theorie. Die Destillation bei vermindertem Druck lieferte 43.7 kg (82.4 % der Theorie) 2-Methylen-1,3-dioxepan (Kp. 60-61°C bei 3 kPa) mit einer gaschromatographischen Reinheit >99 %.

### Beispiel 8:

150.6 g (1.0 mol) 2-Chlormethy1-1,3-dioxepan, 200.0 g (3.0 mol) technisches Kaliumhydroxyd (gepulvert), 8.0 g Polyethylenglycol-1000-dimethylether und 25.5 g (0.25 mol) 3,3-Dimethylbutan-2-ol wurden in 300 ml tert.-Butyl-methylether 6 Stunden unter Rühren und Rückfluß erhitzt. Anschließend wurden die Salze abgetrennt, der tert.-Butyl-methylether abgestilliert. Der Umsatz von 2-Chlormethyl-1,3-dioxepan betrug 97.2 % und die Ausbeute an 2-Methylen-1,3-dioxepan 71.7 %.

### Beispiel 9:

Vorgehen analog Beispiel 8 mit der Abwandlung, daß anstatt 3,3-Dimethylbutan-2-ol 29.0 g (0.25 mol) 2,4-Dimethylpentan-3-ol eingesetzt wurden. Der Umsatz von 2-Chlormethyl-1,3-dioxepan betrug hier 96.8 %, die Ausbeute an 2-Methylen-1,3-dioxepan 77.8 %.

### Beispiel 10:

Vorgehen analog Beispiel 8 mit der Abwandlung, daß anstatt 3,3-Dimethylbutan-2-ol 32.0 g (0.25 mol) 2,6-Dimethylcyclohexanol eingesetzt wurden. Der Umsatz von 2-Chlormethyl-1,3-dioxepan betrug hier 97.4 %, die Ausbeute an 2-Methylen-1,3-dioxepan 87.9 %.

## Patentansprüche

1. Verfahren zur Herstellung von Ketenacetalen durch Dehydrohalogenierung von offenkettigen oder cyclischen 2-Halogenaldehydacetalen mit einem Alkali- oder Erdalkalihydroxid, dadurch gekennzeichnet, daß die Dehydrohalogenierung in einem zweiphasigen System, in Gegenwart einer Katalysatorkombination aus
a) einem Phasentransferkatalysator und
b) einer oder mehreren Verbindungen aus der Gruppe sekundäre Alkohole, tertiäre Alkohole, sekundäre Diole, tertiäre Diole durchgeführt wird.

2. Verfahren nach Anspruch 1, dadurch gekennzeichnet, daß als Edukte 2-Halogenaldehydacetale der Formeln 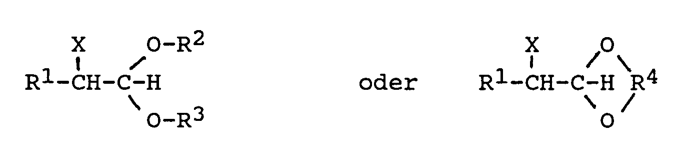 eingesetzt werden, wobei R¹ Wasserstoff, einen geradkettigen, verzweigten oder cyclischen Alkylrest mit 1 bis 18 C-Atomen, einen Alkenylrest, einen Arylrest oder Aralkylrest bedeutet, und
R² und R³ gleich oder verschieden sind und einen Alkylrest mit 1 bis 18 C-Atomen, einen Alkenylrest, einen Arylrest oder Aralkylrest bedeuten, und
R⁴ einen Rest (CR⁵R⁶)m mit m = 2 bis 5 bedeutet, wobei R⁵ und R⁶ gleich oder ungleich sein können und die Bedeutung Wasserstoff, geradkettiger oder verzweigter Alkylrest mit 1 bis 4 C-Atomen, Alkenylrest, Arylrest oder Aralkylrest haben.

3. Verfahren nach Anspruch 2, dadurch gekennzeichnet, daß als Edukte 2-Halogenaldehydacetale eingesetzt werden, wobei X = Cl, R¹ Wasserstoff bedeutet, R² und R³ gleich sind und einen Alkylrest mit 1 bis 4 C-Atomen bedeuten und R⁴ einen -(CH₂)₄-Rest bedeutet.

4. Verfahren nach Anspruch 1 bis 3, dadurch gekennzeichnet, daß als Alkali- oder Erdalkalihydroxide LiOH, NaOH, KOH, CsOH, Ca(OH)₂, Sr(OH)₂, oder Ba(OH)₂, in einem Molverhältnis von 2-Halogenaldehydacetal zu (Erd)alkalihydroxid von 1 : 1 bis 1 : 25, eingesetzt werden.

5. Verfahren nach Anspruch 4, dadurch gekennzeichnet, daß Kaliumhydroxid, in einem Molverhältnis von 2-Halogenaldehydacetal zu Kaliumhydroxid von 1 : 1 bis 1 : 5, eingesetzt wird.

6. Verfahren nach Anspruch 1 bis 5, dadurch gekennzeichnet, daß als Phasentransferkatalysator eine Verbindung aus der Gruppe Polyethylenglykol-dialkylether, Polyethylenglykol-aryl-aralkylether, Polyethylenglykol-alkyl-arylether, Kronenether, Tris-[2-(2-methoxyethoxy)ethyl]-amin, Tetraalkylammoniumsalze, Aralkyl-trialkylammoniumsalze, in einem Gewichtsverhältnis von Phasentransferkatalysator zu 2-Halogenaldehydacetal von 0.001 : 1 bis 1 : 1, eingesetzt wird.

7. Verfahren nach Anspruch 1 bis 6, dadurch gekennzeichnet, daß als sekundäre oder tertiäre Alkohole sowie sekundäre oder tertiäre Diole solche aus der Gruppe Diphenylmethanol, t-Butanol, sec-Butanol, 2-Methyl-2-butanol, 3,3-Dimethyl-2-butanol, 3-Methyl-3-pentanol, 2,4-Dimethyl-3-pentanol, 2,4-Dimethylpentan-2,4-diol, 2,6-Dimethylcyclohexanol, 2,5-Dimethylhexan-2,5-diol, in einem Molverhältnis von sekundärem oder tertiärem Alkohol bzw. Diol zu 2-Halogenaldehydacetal von 0.01 : 1 bis 20 : 1, eingesetzt werden.

8. Verfahren nach Anspruch 1 bis 7, dadurch gekennzeichnet, daß das Molverhältnis von 2-Halogenaldehydacetal zu (Erd)alkalihydroxid 1 : 2 bis 1 : 5 und das Molverhältnis von 2-Halogenaldehydacetal zu Alkohol- bzw. Diol-Komponente 1: 0.01 bis 1 : 0.3 beträgt.

## Claims

1. Process for preparing ketene acetals by dehydrohalogenation of open-chain or cyclic 2-haloaldehyde acetals using an alkali metal or alkaline earth metal hydroxide, characterized in that the dehydrohalogenation is carried out in a two-phase system in the presence of a catalyst combination comprising
a) a phase transfer catalyst and
b) one or more compounds from the group consisting of secondary alcohols, tertiary alcohols, secondary diols and tertiary diols.

2. Process according to Claim 1, characterized in that the starting materials used are 2-haloaldehyde acetals of the formula 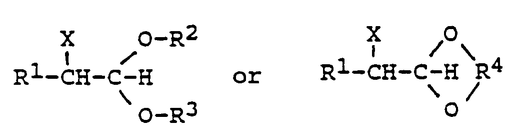 in which R¹ is hydrogen, a straight chain, branched or cyclic alkyl radical having from 1 to 18 carbon atoms, an alkenyl radical, an aryl radical or an aralkyl radical, and
R² and R³ are the same or different and are an alkyl radical having from 1 to 18 carbon atoms, an alkenyl radical, an aryl radical or an aralkyl radical, and
R⁴ is a radical (CR⁵R⁶)ₘ with m = 2 to 5, in which R⁵ and R⁶ may be the same or different and are hydrogen, a straight chain or branched alkyl radical having from 1 to 4 carbon atoms, an alkenyl radical, an aryl radical or an aralkyl radical.

3. Process according to Claim 2, characterized in that the starting materials used are 2-haloaldehyde acetals in which X = Cl, R¹ is hydrogen, R² and R³ are the same and are an alkyl radical having from 1 to 4 carbon atoms and R⁴ is a -(CH₂)₄ radical.

4. Process according to Claims 1 to 3, characterized in that the alkali or alkaline earth metal hydroxides used are LiOH, NaOH, KOH, CsOH, Ca(OH)₂, Sr(OH)₂, or Ba(OH)₂, in a molar ratio of 2-haloaldehyde acetal to alkali or alkaline earth metal hydroxide of from 1 : 1 to 1 : 25.

5. Process according to Claim 4, characterized in that potassium hydroxide is used in a molar ratio of 2-haloaldehyde acetal to potassium hydroxide of from 1 : 1 to 1 : 5.

6. Process according to Claims 1 to 5, characterized in that the phase transfer catalyst used is a compound from the group comprising polyethylene glycol dialkyl ethers, polyethylene glycol aryl aralkyl ethers, polyethylene glycol alkyl aryl ethers, crown ethers, tris-[2-(2-methoxyethoxy)ethyl]amine, tetraalkylammonium salts, aralkyltrialkylammonium salts, in a weight ratio of phase transfer catalyst to 2-haloaldehyde acetal of from 0.001 : 1 to 1 : 1.

7. Process according to Claims 1 to 6, characterized in that the secondary or tertiary alcohols or secondary or tertiary diols used are those from the group comprising diphenylmethanol, t-butanol, sec-butanol, 2-methyl-2-butanol, 3,3-dimethyl-2-butanol, 3-methyl-3-pentanol, 2,4-dimethyl-3-pentanol, 2,4-dimethylpentane-2,4-diol, 2,6-dimethylcyclohexanol, 2,5-dimethylhexane-2,5-diol, in a molar ratio of secondary or tertiary alcohol or diol to 2-haloaldehyde acetal of from 0.01 : 1 to 20 : 1.

8. Process according to Claims 1 to 7, characterized in that the molar ratio of 2-haloaldehyde acetal to alkali or alkaline earth metal hydroxide is from 1 : 2 to 1 : 5 and the molar ratio of 2-haloaldehyde acetal to alcohol or diol component is from 1 : 0.01 to 1 : 0.3.

## Revendications

1. Procédé pour la préparation de cétène-acétals par déshydrohalogénation de 2-halogéno-aldéhydacétals à chaîne ouverte ou cycliques, avec un hydroxyde alcalin ou alcalino-terreux, caractérisé en ce qu'on effectue la déshydrohalogénation dans un système biphasique, en présence d'une combinaison de catalyseurs composé
a) d'un catalyseur de transfert de phases et
b) d'un ou plusieurs composés pris dans le groupe d'alcools secondaires, d'alcools tertiaires, de diols secondaires, de diols tertiaires.

2. Procédé selon la revendication 1, caractérisé en ce qu'on utilise en tant que matières de départ des 2-halogéno-aldéhydacétals de formules 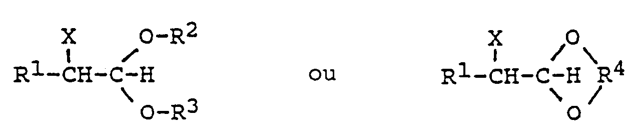 R¹ représentant l'hydrogène, un radical alkyle linéaire, ramifié ou cyclique avec 1 à 18 atomes de carbone, un radical alcényle, un radical aryle ou un radical aralkyle, et
R² et R³ étant identiques ou différents et représentant un radical alkyle avec 1 à 18 atomes de carbone, un radical alcényle, un radical aryle ou un radical aralkyle, et
R⁴ représentant un radical (CR⁵R⁶)ₘ avec m = 2 à 5, R⁵ et R⁶ pouvant être égaux ou inégaux et représenter hydrogène, radical alkyle linéaire ou ramifié avec 1 à 4 atomes de carbone, radical alcényle, radical aryle ou radical aralkyle.

3. Procédé selon la revendication 2, caractérisé en ce qu'on utilise en tant que produits de départ des 2-halogéno-aldéhydacétals, X étant = Cl, R¹ représentant l'hydrogène, R² et R³ étant identiques et un radical alkyle avec 1 à 4 atomes de carbone et R⁴ représentant un radical -(CH₂)₄-.

4. Procédé selon les revendications 1 à 3, caractérisé en ce qu'on utilise en tant qu'hydroxydes alcalins ou alcalino-terreux LiOH, NaOH, KOH, CsOH, Ca(OH)₂, Sr(OH)₂ ou Ba(OH)₂, dans un rapport molaire du 2-halogéno-aldéhydacétal à l'hydroxyde alcalin (alcalino-terreux) de 1:1 à 1:25.

5. Procédé selon la revendication 4, caractérisé en ce qu'on utilise l'hydroxyde de potassium dans un rapport molaire du 2-halogéno-aldéhydacétal à l'hydroxyde de potassium de 1:1 à 1:5.

6. Procédé selon les revendications 1 à 5, caractérisé en ce qu'on utilise en tant que catalyseur de transfert de phases un composé pris dans le groupe comportant le polyéthylèneglycol-dialkyléther, le polyéthylèneglycol-aryl-aralkyléther, le polyéthylèneglycol-alkyl-aryl-éther, les éthers couronne, la tris-[2-(2-méthoxy-éthoxy)éthyl)]-amine, des sels de tétraalkyl-ammonium, des sels d'aralkyl-trialkyl-ammonium, dans un rapport molaire du catalyseur de transfert de phases au 2-halogéno-aldéhydacétal de 0,001:1 à 1:1.

7. Procédé selon les revendications 1 à 6, caractérisé en ce qu'on utilise en tant qu'alcools secondaires ou tertiaires, ainsi que diols secondaires ou tertiaires, ceux pris dans le groupe comportant le diphénylméthanol, le t-butanol, le sec-butanol, le 2-méthyl-2-butanol, le 3,3-diméthyl-2-butanol, le 3-méthyl-3-pentanol, le 2,4-diméthyl-3-pentanol, le 2,4-diméthylpentan-2,4-diol, le 2,6-diméthylcyclohexanol, le 2,5-diméthylhexane-2,5-diol, dans un rapport molaire de l'alcool secondaire ou de l'alcool tertiaire, respectivement du diol, au 2-halogéno-aldéhydacétal de 0,01:1 à 20:1.

8. Procédé selon les revendications 1 à 7, caractérisé en ce que le rapport molaire du 2-halogéno-aldéhydacétal à l'hydroxyde alcalin(alcalino-terreux) est de 1:2 à 1:5 et le rapport molaire du 2-halogéno-aldéhydacétal au composant alcool, respectivement diol, est de 1:0,01 à 1:0,3.
